# EUROPEAN PATENT APPLICATION

(11) **EP 4 137 206 A1**
(43) Date of publication of application: **22.02.2023**
(21) Application number: 21788851.0
(22) Date of filing: 13.04.2021
(51) Int. Cl.: A61P 3/02, A61P 25/00, A61P 25/20, A23L 33/10, A61K 31/194

(54) **AGENT FOR IMPROVING QUALITY OF SLEEP**

(30) Priority: 14.04.2020 JP 2020072192; 20.07.2020 JP 2020123385
(71) Applicant: Nippon Chemiphar Co., Ltd., Tokyo 101-0032 (JP)
(72) Inventor: YAMASAKI Satomi, Tokyo 101-0032 (JP); KAWAGUCHI Kazuhiko, Tokyo 101-0032 (JP); MIZUNO Takanori, Tokyo 101-0032 (JP); SAKURAI Tetsuya, Tokyo 101-0032 (JP)
(74) Representative: V.O.
(86) International application number: PCT/JP2021/015273
(87) International publication number: WO 2021/210565

(57) **Abstract**

Provided is a useful pharmaceutical composition that exhibits effects of improving sleep quality, relieving stress, and reducing fatigue feeling in a mammal (for example, a human) target by ingestion or administration of a composition containing at least one substance selected from the group consisting of citric acid and a salt thereof.

## Description

### Technical Field

The present invention relates to a sleep quality improver (for example, a food composition or a pharmaceutical composition) containing citric acid or a salt thereof as an active ingredient.

This application claims priority based on Japanese Patent Application No. 2020-072192 filed in Japan on April 14, 2020 and Japanese Patent Application No. 2020-123385 filed in Japan on July 20, 2020, the contents of which are incorporated herein by reference.

### Background Art

Sleep is deeply related to rest of the body and mind, growth, repair, or metabolism of the body, reconstruction of memory, and the like, and it is necessary to acquire appropriate sleep in order to live a healthy life. However, for example, according to "National Health and Nutrition Survey Report 2016" of the Ministry of Health, Labour and Welfare of Japan, the ratio of people who cannot take sufficient rest by sleep is 19.7%, and the ratio is significantly increasing in recent years. In an age group of twenties to fifties, the ratio is more than 20%. Due to various circumstances of living environment, it is not always easy to ensure sufficient sleep time, and it is important to improve sleep quality in order to acquire highly satisfactory sleep in a certain sleep time.

So far, okra seed extract (Patent Literature 1), sulfur-containing amino acid having a sulfinyl group (Patent Literature 2), glycine (Patent Literature 3), and the like have been reported as substances that improve sleep quality.

### Citation List

### Patent Literature

Patent Literature 1: JP 2020-048481 A
Patent Literature 2: JP 2019-023179 A
Patent Literature 3: JP 4913410 B2

### Summary of Invention

### Technical Problem

One of objects of the present invention is to provide a sleep quality improver for a mammal (particularly, a human).

One of the objects of the present invention is to provide a stress reliever for a mammal (particularly, a human).

One of the objects of the present invention is to provide a fatigue feeling reducer for a mammal (particularly, a human).

### Solution to Problem

The present inventors made intensive studies for achieving the above objects, and as a result, have found that citric acid or a salt thereof is useful for improving sleep quality, relieving stress, and reducing fatigue feeling of a mammal (particularly, a human), and have completed the present invention.

In one aspect, the present invention provides a sleep quality improver containing citric acid or a salt thereof as an active ingredient.

In one aspect, the present invention provides a stress reliever containing citric acid or a salt thereof as an active ingredient.

In one aspect, the present invention provides a fatigue feeling reducer containing citric acid or a salt thereof as an active ingredient.

### Advantageous Effects of Invention

A composition provided by the present invention is useful for improving sleep quality, relieving stress, reducing fatigue feeling, and the like of a mammal (particularly, a human).

### Brief Description of Drawings

Fig. 1 is a graph illustrating results of evaluating sleep quality of a subject who has received a citrate containing citric acid, a potassium citrate monohydrate, and a sodium citrate dihydrate or a placebo on the basis of St. Mary's hospital sleep questionnaire (results of evaluating how many times a subject awoke).
Fig. 2 is a graph illustrating results of evaluating sleep quality of a subject who has received a citrate containing citric acid, a potassium citrate monohydrate, and a sodium citrate dihydrate or a placebo on the basis of St. Mary's hospital sleep questionnaire (results of evaluating sleep).
Fig. 3 is a graph illustrating results of evaluating sleep quality of a subject who has received a citrate containing citric acid, a potassium citrate monohydrate, and a sodium citrate dihydrate or a placebo on the basis of St. Mary's hospital sleep questionnaire (results of evaluating how well a subject could sleep).
Fig. 4 is a graph illustrating results of evaluating sleep quality of a subject who has received a citrate containing citric acid, a potassium citrate monohydrate, and a sodium citrate dihydrate or a placebo by measuring brain waves (the number of times of intermediate awakening).
Fig. 5 is a graph illustrating results of evaluating sleep quality of a subject who has received a citrate containing citric acid, a potassium citrate monohydrate, and a sodium citrate dihydrate or a placebo by measuring brain waves (the number of times of intermediate awakening during two hours before getting-up).
Fig. 6 is a graph illustrating results of evaluating sleep quality of a subject who has received a citrate containing citric acid, a potassium citrate monohydrate, and a sodium citrate dihydrate or a placebo by measuring brain waves (a ratio of non-REM sleep stage 1 to total sleep time).
Fig. 7 is a graph illustrating results of evaluating sleep quality of a subject who has received a citrate containing citric acid, a potassium citrate monohydrate, and a sodium citrate dihydrate or a placebo by measuring brain waves (time of non-REM sleep stage 1).
Fig. 8 is a graph illustrating results of evaluating sleep quality of a subject who has received a citrate containing citric acid, a potassium citrate monohydrate, and a sodium citrate dihydrate or a placebo by measuring brain waves (a ratio of non-REM sleep stage 3 to total sleep time).
Fig. 9 is a graph illustrating results of evaluating sleep quality of a subject who has received a citrate containing citric acid, a potassium citrate monohydrate, and a sodium citrate dihydrate or a placebo by measuring brain waves (time of non-REM sleep stage 3).
Fig. 10 is a graph illustrating results of evaluating sleep quality of a subject who has received a citrate containing citric acid, a potassium citrate monohydrate, and a sodium citrate dihydrate or a placebo on the basis of St. Mary's hospital sleep questionnaire (degree of refreshness at the time of getting-up).
Fig. 11 illustrates results of evaluating a mood/emotion of a subject (male or female) who has received a citrate containing citric acid, a potassium citrate monohydrate, and a sodium citrate dihydrate or a placebo on the basis of POMS2 (mood profile test). Specifically, Fig. 11 illustrates a change amount of a POMS2 score of a subject after a test of administering a citrate containing citric acid, a potassium citrate monohydrate, and a sodium citrate dihydrate or a placebo from a POMS2 score of the subject before the test.
Fig. 12 illustrates results of evaluating a mood/emotion of a subject (female) who has received a citrate containing citric acid, a potassium citrate monohydrate, and a sodium citrate dihydrate or a placebo on the basis of POMS2 (mood profile test). Specifically, Fig. 12 illustrates a change amount of a POMS2 score of a subject after a test of administering a citrate containing citric acid, a potassium citrate monohydrate, and a sodium citrate dihydrate or a placebo from a POMS2 score of the subject before the test.
Fig. 13 is a graph illustrating a pulse rate of a subject before performing a test of administering a citrate containing citric acid, a potassium citrate monohydrate, and a sodium citrate dihydrate or a placebo.
Fig. 14 is a graph illustrating a pulse rate of a subject after performing a test of administering a citrate containing citric acid, a potassium citrate monohydrate, and a sodium citrate dihydrate or a placebo.
Fig. 15 is a graph illustrating a change amount of a pulse rate of a subject after a test of administering a citrate containing citric acid, a potassium citrate monohydrate, and a sodium citrate dihydrate or a placebo from a pulse rate of the subject before the test.
Fig. 16 illustrates results of evaluating sleep quality of a subject (male or female) who has received a citrate containing citric acid, a potassium citrate monohydrate, and a sodium citrate dihydrate or a placebo on the basis of an OSA sleep questionnaire. Specifically, Fig. 16 illustrates a change amount of an OSA score of a subject after a test of administering a citrate containing citric acid, a potassium citrate monohydrate, and a sodium citrate dihydrate or a placebo from an OSA score of the subject before the test.
Fig. 17 illustrates results of evaluating sleep quality of a subject (female) who has received a citrate containing citric acid, a potassium citrate monohydrate, and a sodium citrate dihydrate or a placebo on the basis of an OSA sleep questionnaire. Specifically, Fig. 17 illustrates a change amount of an OSA score of a subject after a test of administering a citrate containing citric acid, a potassium citrate monohydrate, and a sodium citrate dihydrate or a placebo from an OSA score of the subject before the test.
Fig. 18 illustrates results of evaluating fatigue feeling of a subject (male or female) who has received a citrate containing citric acid, a potassium citrate monohydrate, and a sodium citrate dihydrate or a placebo on the basis of VAS.
Fig. 19 illustrates results of evaluating fatigue feeling of a subject (female) who has received a citrate containing citric acid, a potassium citrate monohydrate, and a sodium citrate dihydrate or a placebo on the basis of VAS.

### Description of Embodiments

The present invention provides a sleep quality improver, a stress reliever, and a fatigue feeling reducer each containing citric acid or a salt thereof as an active ingredient.

The sleep quality improver, the stress reliever, and the fatigue feeling reducer provided by the present invention can be a food composition or a pharmaceutical composition.

Examples of citric acid include, but are not limited to, a hydrate of citric acid (for example, a citric acid monohydrate (C₆H₈O₇·H₂O)) and anhydrous citric acid.

Preferable examples of the salt of citric acid include, but not limited to, a food-acceptable or pharmaceutically acceptable salt of citric acid, and examples thereof include a sodium salt of citric acid and a potassium salt of citric acid.

Examples of the sodium salt of citric acid include, but are not limited to, monosodium citrate, disodium citrate, trisodium citrate (trisodium citrate may be referred to simply as sodium citrate in the present specification), and sodium ferrous citrate. The sodium salt of citric acid may be a hydrate thereof, and may be, for example, a sodium citrate dihydrate (C₆H₅Na₃O₇·2H₂O).

Examples of the potassium salt of citric acid include, but are not limited to, monopotassium citrate, dipotassium citrate, and tripotassium citrate (tripotassium citrate may be referred to simply as potassium citrate in the present specification). The potassium salt of citric acid may be a hydrate thereof, and may be, for example, a potassium citrate monohydrate (C₆H₅K₃O₇·H₂O).

The salt of citric acid may be a hydrate thereof or a mixture of different salts or hydrates thereof. When the salt of citric acid is a mixture, a mixing ratio between the salts of citric acid constituting the mixture can be appropriately set by a person skilled in the art, and when the mixture includes two types of salts of citric acid, a molar ratio thereof can be set to 0.01 to 100. The mixing ratio may be about 1 : 1 in a molar ratio.

In one embodiment, the salt of citric acid contained in the composition provided by the present invention (for example, a sleep quality improver, a stress reliever, or a fatigue feeling reducer) can be a mixture of a sodium salt of citric acid and a potassium salt of citric acid. A mixing ratio between the number of moles of the sodium salt of citric acid and the number of moles of the potassium salt of citric acid in the mixture can be appropriately set by a person skilled in the art, and is preferably 0.85 : 1.15 to 1.15 : 0.85, more preferably 0.90 : 1.10 to 1.10 : 0.90, still more preferably 0.95 : 1.05 to 1.05 : 0.95, further still more preferably 0.99 : 1.01 to 1.01 : 0.99, and particularly preferably 1 : 1.

In one embodiment, the salt of citric acid contained in the compositions provided by the present invention (for example, a sleep quality improver, a stress reliever, or a fatigue feeling reducer) can be a mixture of a sodium citrate dihydrate (C₆H₅Na₃O₇·2H₂O) and a potassium citrate monohydrate (C₆H₅K₃O₇·H₂O). A mixing ratio between the number of moles of the sodium citrate dihydrate (C₆H₅Na₃O₇·2H₂O) and the potassium citrate monohydrate (C₆H₅K₃O₇·H₂O) in the mixture can be appropriately set by a person skilled in the art, and is preferably 0.85 : 1.15 to 1.15 : 0.85, more preferably 0.90 : 1.10 to 1.10 : 0.90, still more preferably 0.95 : 1.05 to 1.05 : 0.95, further still more preferably 0.99 : 1.01 to 1.01 : 0.99, and particularly preferably 1 : 1.

In one embodiment, the composition provided by the present invention (for example, a sleep quality improver, a stress reliever, or a fatigue feeling reducer) contains at least one substance selected from the group consisting of citric acid, a sodium salt of citric acid, and a potassium salt of citric acid.

The composition provided by the present invention (for example, a sleep quality improver, a stress reliever, or a fatigue feeling reducer) may contain citric acid, a sodium salt of citric acid, and a potassium salt of citric acid. The citric acid, the sodium salt of citric acid, and the potassium salt of citric acid can be active ingredients. The citric acid, the sodium salt of citric acid, and the potassium salt of citric acid can be a mixture, and a mixing ratio among the number of moles of the citric acid, the number of moles of the sodium salt of citric acid, and the number of moles of the potassium salt of citric acid in the mixture can be appropriately set by a person skilled in the art. For example, as for a molar ratio among the potassium salt of citric acid, the sodium salt of citric acid, and the citric acid, the number of moles of the sodium salt of citric acid and the number of moles of the citric acid can be 0.01 to 100 and 0.01 to 100, respectively, with respect to 1 mole of the potassium salt of citric acid. The mixing ratio may be about 2 : 2 : 1 in a molar ratio.

In one embodiment, the composition provided by the present invention (for example, a sleep quality improver, a stress reliever, or a fatigue feeling reducer) contains a mixture of anhydrous citric acid, a sodium salt of citric acid, and a potassium salt of citric acid. A mixing ratio among the number of moles of the anhydrous citric acid, the number of moles of the sodium salt of citric acid, and the number of moles of the potassium salt of citric acid in the mixture can be appropriately set by a person skilled in the art, can be, for example, 1 : 1 to 3 : 1 to 3, 1 : 1.5 to 2.5 : 1.5 to 2.5, and is preferably 1 : 1.7 to 2.3 : 1.7 to 2.3, more preferably 1 : 1.9 to 2.1 : 1.9 to 2.1, still more preferably 1 : 1.95 to 2.05 : 1.95 to 2.05, and particularly preferably 1 : 2 : 2.

In one embodiment, the composition provided by the present invention (for example, a sleep quality improver, a stress reliever, or a fatigue feeling reducer) contains a mixture of anhydrous citric acid, a sodium citrate dihydrate (C₆H₅Na₃O₇·2H₂O), and a potassium citrate monohydrate (C₆H₅K₃O₇·H₂O). A mixing ratio among the number of moles of anhydrous citric acid, the number of moles of the sodium citrate dihydrate (C₆H₅Na₃O₇·2H₂O), and the number of moles of the potassium citrate monohydrate (C₆H₅K₃O₇·H₂O) in the mixture can be appropriately set by a person skilled in the art, can be 1 : 1 to 3 : 1 to 3, 1 : 1.5 to 2.5 : 1.5 to 2.5, and is preferably 1 : 1.7 to 2.3 : 1.7 to 2.3, more preferably 1 : 1.9 to 2.1 : 1.9 to 2.1, still more preferably 1 : 1.95 to 2.05 : 1.95 to 2.05, and particularly preferably 1 : 2 : 2.

In the present specification, when referring to the weight of citric acid or a salt thereof (for example, a potassium citrate monohydrate (C₆H₅K₃O₇·H₂O) or a sodium citrate dihydrate (C₆H₅Na₃O₇·2H₂O)), the weight can be on a dry basis.

The composition provided by the present invention (for example, a sleep quality improver, a stress reliever, or a fatigue feeling reducer) can exhibit an action of improving sleep quality, an action of relieving stress, and/or an action of reducing fatigue feeling.

In one embodiment, the action exhibited by the composition provided by the present invention (for example, an action of improving sleep quality, an action of relieving stress, or an action of reducing fatigue feeling) can be confirmed when the composition provided by the present invention is ingested or taken in comparison with a case where the composition provided by the present invention is not ingested or taken.

In one embodiment, the action exhibited by the composition provided by the present invention (for example, an action of improving sleep quality, an action of relieving stress, or an action of reducing fatigue feeling) can be confirmed when the composition provided by the present invention is ingested or taken in comparison with a condition before the composition provided by the present invention is ingested or taken.

In a case where the composition provided by the present invention contains a sodium salt of citric acid and a potassium salt of citric acid, when a molar ratio between the sodium salt of citric acid and the potassium salt of citric acid is 1 : 0.8 to 1.2, 1 : 0.9 to 1.1, or 1 : 1, the composition can have an advantage that the composition has little or no influence on a blood pressure of a mammal (for example, a human) that has ingested or taken the composition.

For example, when a mixture containing sodium citrate, potassium citrate, and citric acid at 2 : 2 : 1 was orally administered to a rat that had been subjected to one kidney removal-deoxycorticosterone acetate (DOCA) loading for three consecutive weeks (2000 mg/kg/day), an increase in blood pressure due to the administration was not observed. Meanwhile, when saline was administered for three weeks, a significant increase in blood pressure was observed after the administration for three weeks in comparison with the rat before the administration. It was considered that the sodium concentration in blood of the sodium citrate, potassium citrate, and citric acid mixture (mixing ratio 2 : 2 : 1) administration group tended to be higher than that of the saline administration group. From such a fact, it is supported that when the molar ratio between the sodium salt of citric acid and the potassium salt of citric acid is 1 : 0.8 to 1.2, 1 : 0.9 to 1.1, or 1 : 1, an influence on a blood pressure of a mammal (for example, a human) that has ingested or taken a mixture containing the sodium salt of citric acid and the potassium salt of citric acid (for example, a mixture of sodium citrate, potassium citrate, and citric acid (mixing ratio 2 : 2 : 1)) is small.

The composition provided by the present invention (for example, a sleep quality improver, a stress reliever, or a fatigue feeling reducer) can be ingested by or administered to a mammal (for example, a human).

In the present specification, "mammal" includes an animal classified as Mammalia, and a human is particularly preferable.

In general, it is known that human sleep transitions among states of shallow sleep (non-REM sleep stage 1 and stage 2), slow wave sleep (also referred to as deep sleep, non-REM sleep stage 3 and stage 4) (shallow sleep and slow wave sleep may be referred to as non-REM sleep), and REM sleep, and it is also known that a human may awake during sleep and may transition to a sleep state again (may be referred to as intermediate awakening). In the present specification, a "sleep cycle" refers to a period from an end of a certain REM sleep to an end of the next REM sleep. A "first sleep cycle" refers to a first sleep cycle after falling asleep.

The "sleep quality" can be generally evaluated by, for example, an objective index such as the number of times and time of intermediate awakening, sleep efficiency, presence or absence of early morning awakening, non-REM sleep time extension, time until entering non-REM sleep and REM sleep, time until entering slow wave sleep, the number of times and time of slow wave sleep, a ratio of time of slow wave sleep to total sleep time, sleep time, sleep latent time (time until falling asleep), or a delta power value during non-REM sleep (index of the depth of sleep), or a subject's relatively subjective index such as how easily a subject falls asleep, refreshed feeling at the time of awakening, good sleep feeling, dreaming (for example, whether or not to have a nightmare or the number of times of dreaming), the degree of satisfaction of sleep, the degree of daytime drowsiness, or the degree of fatigue feeling. The subjective index can be evaluated by, for example, St. Mary's hospital sleep questionnaire, POMS2, OSA sleep questionnaire, or VAS.

The "improvement in sleep quality" provided by the present invention can be evaluated as, for example, suppression of intermediate awakening (for example, a decrease in the number of times of intermediate awakening), promotion of slow wave sleep (for example, an increase in time of slow wave sleep (for example, non-REM sleep stage 3) or an increase in ratio of time of slow wave sleep (for example, non-REM sleep stage 3) to total sleep time), an increase in delta power value of the first sleep cycle), suppression of early morning awakening (for example, a decrease in the number of times of intermediate awakening during two hours before getting-up), an increase in time of slow wave sleep, an increase in ratio of time of slow wave sleep to total sleep time, promotion of formation of deep sleep (for example, an increase in delta power value (for example, a delta power value of the first sleep cycle)), improvement in good sleep feeling, improvement in the degree of refreshness at the time of getting-up, a state of mood, or reduction of fatigue feeling in comparison with sleep quality before ingestion of the composition of the present invention (for example, a food composition or a pharmaceutical composition) or in comparison with a control or a placebo.

The number of times of intermediate awakening, the REM sleep, the non-REM sleep, the slow wave sleep, the delta power value, and the like can be evaluated by a known method, and can be evaluated, for example, by measuring brain waves using an electroencephalograph.

In one embodiment, the "improvement in sleep quality" is an increase in ratio of time of slow wave sleep (for example, non-REM sleep stage 3) to total sleep time. When a state of slow wave sleep (for example, non-REM sleep stage 3) appears a plurality of times during sleep, the time of slow wave sleep (for example, non-REM sleep stage 3) can be a total time obtained by summing up time for each state of slow wave sleep (for example, non-REM sleep stage 3) that appears a plurality of times.

In one embodiment, the ratio of time of slow wave sleep to total sleep time is preferably increased by 10% to 50%, and more preferably increased by 15% to 35% in comparison with the ratio before ingestion of the composition of the present invention or in comparison with a control or a placebo.

In one embodiment, the "improvement in sleep quality" is a decrease in the number of times of intermediate awakening.

In one embodiment, as for the number of times of intermediate awakening, the number of times determined to be awakening from measured brain waves is preferably decreased by 5 to 30%, and more preferably decreased by 10 to 20% in comparison with the number of times of intermediate awakening before ingestion of the composition of the present invention or in comparison with a control or a placebo.

In addition, in one embodiment, the number of times of intermediate awakening may be evaluated by the number of times of actual recognition of awakening during a sleep period. The number of times of intermediate awakening is preferably decreased by one time in comparison with the number of times of intermediate awakening before ingestion of the composition of the present invention or in comparison with a control or a placebo, and is particularly preferably 0.

In one embodiment, the "improvement in sleep quality" is improvement in good sleep feeling, and the improvement in good sleep feeling may be evaluated by using a fact that a subject subjectively feels that the subject has good sleep in comparison with sleep quality before ingestion of the composition of the present invention or in comparison with a control or a placebo as an index.

In one embodiment, the "improvement in sleep quality" is promotion of formation of deep sleep, and the promotion of formation of deep sleep may be evaluated by using a fact that a subject subjectively feels that the subject has deep sleep in comparison with sleep quality before ingestion of the composition of the present invention or in comparison with a control or a placebo as an index, or may be evaluated by using an increase in delta power value as an index.

In one embodiment, the "improvement in sleep quality" is improvement in the degree of refreshness at the time of getting-up, and the improvement in the degree of refreshness may be evaluated by using a fact that a subject subjectively feels that the degree of refreshness at the time of getting-up is improved in comparison with sleep quality before ingestion of the composition of the present invention or in comparison with a control or a placebo as an index.

The degree of refreshness at the time of getting-up can be rephrased as, for example, refreshed feeling at the time of awakening, awakening feeling at the time of getting-up, drowsiness at the time of getting-up, awakening refreshed, awakening comfortable, crisp awakening, awakening fresh, good feeling awakening, pleasant awakening, brisk awakening, fresh awakening, relief awakening, good awakening, awakening fine, awakening good, awakening great, smooth awakening, awakening light, healthy awakening, awakening refreshing, pleasant awakening, light awakening, awakening vigorous, awakening cheerful, bright awakening, or high quality awakening.

In one embodiment, by ingesting or taking the composition provided by the present invention, for example, for a period of one or two days, for a period of five or more days, or for a period of one or two weeks, a subject can improve his/her sleep quality.

In the present specification, excessive urination in the nighttime refers to a state in which a nighttime urine volume increases, and for example, refers to a state in which a urine volume during sleep in the nighttime exceeds 33% of a total urine volume in one day in an elderly person, and a state in which a urine volume during sleep in the nighttime exceeds 200 of a total urine volume in one day in a young person. Nocturia refers to a symptom in which a subject must get up one or more times during sleep to urinate. Nocturia can be nocturia due to excessive urination in the nighttime.

When the number of times of urination in the nighttime decreases, intermediate awakening can be suppressed. Intermediate awakening can cause sleep disorder and deteriorate sleep quality. In addition, even if urination is not reached, if the production amount of nighttime urine increases, awakening can be caused by a desire to urinate, sleep disorder can be caused, and sleep quality can be deteriorated.

Therefore, the present invention provides, in one embodiment, a composition for suppressing sleep disorder caused by frequent urination (for example, nocturia or nocturia due to excessive urination in the nighttime), provides, in one embodiment, a composition for suppressing intermediate awakening, and provides, in one embodiment, a composition for improving sleep quality (for example, a composition for improving sleep quality by suppressing a desire to urinate in the nighttime or reducing the number of times of urination in the nighttime).

In one embodiment, the "decrease in the number of times of urination" can be evaluated by comparison with the number of times of urination before ingestion of the composition of the present invention or by comparison with a control or a placebo.

The composition provided by the present invention can also relieve stress (physical or mental stress) in a target (for example, a human) that has ingested or taken the composition. The relief of stress may be evaluated by using a state of mood (for example, anger, hostility, confusion, bafflement, depression, decline, fatigue, inertia, tension, anxiety, activeness, vitality, effectiveness, or total mood disturbance) in comparison with stress before ingestion of the composition of the present invention or in comparison with a control or a placebo as an index

When ataxia occurs in the autonomic nervous system due to physical or mental stress, an enhanced mental state is caused, rapid falling asleep (sleep inducing) is prevented, and shallow sleep is caused. By ingesting or taking the composition provided by the present invention, a subject can relieve his/her stress.

The stress can be mental stress or physical stress, and can be distortion generated in the mind or body in order to adapt to external stimulus (stressor) applied to the mind or body. Examples of the stressor include, but are not limited to, "physical stressor" (heat, cold, noise, congestion, or the like), "chemical stressor" (pollutant, drug, oxygen deficiency/excess, carbon monoxide, or the like), and "psychological/social stressor" (problems in human relation and work, home problem, or the like).

Examples of the mental stress that can be caused by a stressor include a decrease in activeness, irritation, anxiety, and depression (decline in mood or decrease in interest/concern). Meanwhile, examples of the physical stress include various symptoms such as pain in joints of the body, headache, stiff shoulder, low back pain, eye fatigue, palpitation and shortness of breath, stomach pain, a decrease in appetite, and constipation and diarrhea. Whether or not the mental stress has been relieved may be evaluated, for example, by a fact that one or more items, two or more items, three or more items, preferably four or more items, and more preferably five or more items are improved among seven items of anger-hostility; confusion-bafflement; depression-decline; fatigue-inertia; tension-anxiety; activeness-vitality; and friendship and a TMD score indicating a negative mood state in a mood profile test such as POMS2 described in Examples.

In addition, a stressor makes the sympathetic nerve active and can change the autonomic nerve activity. For example, it is known that a pulse rate increases when stress is loaded, but the parasympathetic nerve becomes active and the pulse rate decreases when the stress is relieved. Therefore, whether or not the physical stress has been relieved may be evaluated by measuring a pulse rate (heart rate).

By ingesting or taking the composition provided by the present invention, for example, for a period of one or two days, for a period of five or more days, or for a period of one or two weeks, a subject can relieve or reduce his/her stress.

The composition provided by the present invention can also reduce fatigue feeling of a target(for example, a human) that has ingested or taken the composition. The reduction in fatigue feeling may be evaluated by using a fact that a subject subjectively feels that fatigue feeling is reduced in comparison with fatigue feeling before ingestion of the composition of the present invention or in comparison with a control or a placebo as an index, or may be evaluated by using VAS or the like. By ingesting or taking the composition provided by the present invention, for example, for a period of one or two days or for a period of one or two weeks, a subject can reduce his/her fatigue feeling.

The compositions provided by the present invention can be food or medicine and can be produced by a method known in the field of food or medicine.

In the present specification, examples of the food include, but are not limited to, a food for specified health use, a functionally labeled food, a food for hospital patients, and a supplement. The form of the food is not particularly limited as long as the food contains citric acid and/or a salt thereof and is in an orally ingestible form, and may be in a form of normal food and beverage, or may be formulated and provided as a formulation suitable for oral administration, for example, a formulation such as a tablet, a capsule, a granule, a jelly, or a liquid (for example, a suspending agent or a drink agent). In the present specification, as for the compositions and production methods of these formulations, a formulation technique known per se in the field of pharmaceutical formulation technique (hereinafter, also referred to as a pharmaceutical field) can be applied to the food formulations.

The composition provided by the present invention may be provided as a form of normal food and beverage without being formulated.

In one embodiment, the composition provided by the present invention can be provided as a normal food and beverage containing citric acid and/or a salt thereof. In this embodiment, a person skilled in the art can appropriately produce the food and beverage, and for example, can produce the food and beverage by blending citric acid and/or a salt thereof with a food material.

Examples of the food and beverage include, but are not limited to, a liquid, emulsified, or pasty food such as a beverage, soy sauce, milk, yogurt, or miso (fermented soybean paste), or dressing; a semisolid food such as jelly or gummy candy; a solid food such as candy, gum, tofu (soybean curd), or a supplement; and a powdery food. Seasoning is also an example of the composition provided by the present invention.

Examples of the beverage include a fruit juice/fruit beverage, a coffee beverage, an oolong tea beverage, a green tea beverage, a black tea beverage, a barley tea beverage, a vegetable beverage, a carbonated beverage as a soft drink, a fruit extract-containing beverage, a vegetable extract-containing juice, near water (soft drink with minute amounts of flavoring, or the like), a sport beverage, a diet beverage, and an alcoholic beverage.

The beverage can contain additives such as an antioxidant, a fragrance, various esters, organic acids, organic acid salts, inorganic acids, inorganic acid salts, inorganic salts, pigments, an emulsifier, a preservative, a seasoning, a sweetener, an acidulant, fruit juice extracts, vegetable extracts, flower honey extracts, a pH regulator, and a quality stabilizer singly or in combination thereof.

Concentrated beverages (liquids) of the above beverages, which can be brought into a normal drinking state by being diluted with a diluting material such as water, carbonated water, or an alcohol aqueous solution, for example, a concentrated beverage, a cocktail conc., and a syrup are also examples of the composition provided by the present invention.

Concentrated beverages (granules) of the above beverages, which can be brought into a normal drinking state by being dissolved in water, carbonated water, an alcohol aqueous solution, or the like are also examples of the composition provided by the present invention.

The ingestion or administration amount of citric acid and/or a salt thereof by the composition provided by the present invention is appropriately determined according to the type of the active ingredient, an ingestion or administration method, the age, weight, sex, symptom, sensitivity to the active ingredient, and the like of an ingestion or an administration target.

In one embodiment, the composition provided by the present invention is ingested or taken by a female.

In one embodiment, the composition provided by the present invention is ingested or taken by a male or a female.

In one embodiment, when the composition provided by the present invention is a food, the composition is ingested by a non-pathological person who wants to improve his/her sleep quality.

In one embodiment, when the composition provided by the present invention is a food, the composition is ingested by a non-pathological person who is experiencing mental and/or physical stress.

In one embodiment, when the composition provided by the present invention is a food, the composition is ingested by a non-pathological person who wants to reduce his/her fatigue feeling.

In one embodiment, the food or medicine provided by the present invention has a unit packaging form or a unit administration form per meal, and may contain citric acid and/or a salt thereof in an amount of 1 mg or more, 10 mg or more, 50 mg or more, 100 mg or more, 200 mg or more, 400 mg or more, 500 mg or more, 1 g or more, 3 g or more, or 5 g or more, for example, in an amount of 50 mg or more and 10 g or less, 100 mg or more and 10 g or less, 200 mg or more and 10 g or less, 400 mg or more and 10 g or less, 500 mg or more and 10 g or less, 1 g or more and 10 g or less, 3 g or more and 10 g or less, 5 g or more and 10 g or less, 50 mg or more and 5 g or less, 100 mg or more and 5 g or less, 200 mg or more and 5 g or less, 400 mg or more and 5 g or less, 500 mg or more and 5 g or less, 1 g or more and 5 g or less, 3 g or more and 5 g or less, 50 mg or more and 3 g or less, 100 mg or more and 3 g or less, 200 mg or more and 3 g or less, 400 mg or more and 3 g or less, 500 mg or more and 3 g or less, 1 g or more and 3 g or less, 50 mg or more and 1 g or less, 100 mg or more and 1 g or less, 200 mg or more and 1 g or less, 400 mg or more and 1 g or less, 50 mg or more and 500 mg or less, 100 mg or more and 500 mg or less, 200 mg or more and 500 mg or less, 400 mg or more and 500 mg or less, 50 mg or more and 200 mg or less, 100 mg or more and 200 mg or less, 10 mg or more and 500 mg or less, 10 mg or more and 200 mg or less, 10 mg or more and 100 mg or less, 10 mg or more and 50 mg or less, 1 mg or more and 500 mg or less, 1 mg or more and 200 mg or less, 1 mg or more and 100 mg or less, 1 mg or more and 50 mg or less, or 1 mg or more and 10 mg or less. Such a unit packaging form or unit administration form may be ingested or taken once a day, twice a day, or three times a day. In a case of twice a day, such a unit packaging form or unit administration form may be ingested or taken after breakfast and before going to bed.

In one embodiment, the food or medicine provided by the present invention has a unit packaging form or a unit administration form per meal, and may contain citric acid, a sodium salt of citric acid, and a potassium salt of citric acid in an amount of 1 mg or more, 10 mg or more, 50 mg or more, 100 mg or more, 200 mg or more, 300 mg or more, 400 mg or more, 500 mg or more, 1 g or more, 3 g or more, or 5 g or more, for example, in an amount of 50 mg or more and 10 g or less, 100 mg or more and 10 g or less, 200 mg or more and 10 g or less, 400 mg or more and 10 g or less, 500 mg or more and 10 g or less, 1g or more and 10 g or less, 3g or more and 10 g or less, 5g or more and 10 g or less, 50 mg or more and 5 g or less, 100 mg or more and 5 g or less, 200 mg or more and 5 g or less, 400 mg or more and 5 g or less, 500 mg or more and 5 g or less, 1 g or more and 5 g or less, 3 g or more and 5 g or less, 200 mg or more and 3 g or less, 300 mg or more and 3 g or less, 400 mg or more and 3 g or less, 500 mg or more and 3 g or less, 200 mg or more and 2 g or less, 300 mg or more and 2 g or less, 400 mg or more and 2 g or less, 500 mg or more and 2 g or less, 1 mg or more and 1 g or less, 100 mg or more and 1 g or less, 200 mg or more and 1 g or less, 300 mg or more and 1 g or less, 400 mg or more and 1 g or less, 500 mg or more and 1 g or less, 10 mg or more and 500 mg or less, 10 mg or more and 200 mg or less, 10 mg or more and 100 mg or less, 10 mg or more and 50 mg or less, 1 mg or more and 500 mg or less, 1 mg or more and 200 mg or less, 1 mg or more and 100 mg or less, 1 mg or more and 50 mg or less, or 1 mg or more and 10mg or less. Such a unit packaging form or unit administration form may be ingested or taken once a day, twice a day, or three times a day. In a case of twice a day, such a unit packaging form or unit administration form may be ingested or taken after breakfast and before going to bed.

In one embodiment, for example, a food for specified health use, a nutritional supplement, a functionally labeled food, or a food for hospital patients may contain anhydrous citric acid, a potassium citrate monohydrate (C₆H₅K₃O₇·H₂O), and a sodium citrate dihydrate (C₆H₅Na₃O₇·2H₂O) in total in an amount of 1/3 of 1 to 3 g per serving of the food. When a food for specified health use, a nutritional supplement, a functionally labeled food, a food for hospital patients, or a supplement is provided as a tablet, for example, 300 mg to 600 mg of one tablet may contain 70 to 95% by weight of citric acid (for example, anhydrous citric acid), a sodium salt of citric acid (for example, sodium citrate dihydrate (C₆H₅Na₃O₇·2H₂O)) and a potassium salt of citric acid (for example, potassium citrate monohydrate (C₆H₅K₃O₇·H₂O)).

A period of ingestion or administration of the composition provided by the present invention (for example, food or medicine) is not particularly limited, and can be, for example, one day or more, two days or more, three days or more, five days or more, one week or more, two weeks or more, four weeks or more, one week, or two weeks.

Since the composition provided by the present invention can exhibit an action of improving sleep quality, an action of relieving stress, and/or an action of reducing fatigue feeling, the composition provided by the present invention can be a composition for improving sleep quality (for example, a composition for suppressing intermediate awakening, a composition for suppressing early morning awakening, a composition for improving good sleep feeling, a composition for promoting slow wave sleep, or a composition for improving awakening feeling at the time of getting-up), a composition for relieving stress, and/or a composition for reducing fatigue feeling.

In another aspect, the present invention provides use of citric acid and/or a salt thereof for improving sleep quality (for example, suppression of intermediate awakening, suppression of early morning awakening, improvement in good sleep feeling, promotion of slow wave sleep, or improvement in awakening feeling at the time of getting-up), for relieving stress, and/or for reducing fatigue feeling.

Other examples of the embodiments provided by the present invention include the following (1) to (2-33):
(1) Use of citric acid or a salt thereof for producing a composition for improving sleep quality;
(2) The use according to (1), in which the improvement in sleep quality is suppression of intermediate awakening;
(3) The use according to (1) or (2), in which the improvement in sleep quality is suppression of early morning awakening;
(4) The use according to any one of (1) to (3), in which the improvement in sleep quality is improvement in good sleep feeling;
(5) The use according to any one of (1) to (4), in which the improvement in sleep quality is promotion of slow wave sleep;
(6) The use according to any one of (1) to (5), in which the improvement in sleep quality is an increase in ratio of time of non-REM sleep stage 3 to total sleep time;
(7) The use according to any one of (1) to (6), in which the improvement in sleep quality is promotion of slow wave sleep that decreases a ratio of time of stage 1 and increases a ratio of time of stage 3 in non-REM sleep;
(8) The use according to any one of (1) to (7), in which the improvement in sleep quality is improvement in sleep quality in a first sleep cycle;
(9) The use according to any one of (1) to (8), in which the improvement in sleep quality is an increase in delta power value in a first sleep cycle;
(10) The use according to any one of (1) to (9), in which the improvement in sleep quality is promotion of formation of deep sleep in a first sleep cycle;
(11) The use according to any one of (1) to (10), in which the improvement in sleep quality is suppression of sleep disorder associated with frequent urination;
(12) The use according to any one of (1) to (11), in which the improvement in sleep quality is suppression of intermediate awakening associated with frequent urination;
(13) The use according to any one of (1) to (12), in which the improvement in sleep quality is improvement in refreshed feeling at the time of getting-up;
(14) The use according to any one of (1) to (13), in which the improvement in sleep quality is improvement in mental state;
(15) Use of citric acid or a salt thereof for producing a stress relieving composition;
(16) Use of citric acid or a salt thereof for producing a mental stress relieving composition;
(17) Use of citric acid or a salt thereof for producing a fatigue feeling reducing composition;
(18) The use according to any one of (1) to (17), in which the composition contains citric acid as an active ingredient;
(19) The use according to any one of (1) to (18), in which the composition contains an alkali metal salt of citric acid as an active ingredient;
(20) The use according to (19), in which the alkali metal salt of citric acid is a sodium salt of citric acid or a hydrate thereof, or a potassium salt of citric acid or a hydrate thereof;
(21) The use according to (20), in which the sodium salt of citric acid is a sodium citrate dihydrate (C₆H₅Na₃O₇·2H₂O);
(22) The use according to (20), in which the potassium salt of citric acid is a potassium citrate monohydrate (C₆H₅K₃O₇·H₂O);
(23) The use according to any one of (1) to (22), in which the citric acid or a salt thereof is a mixture of a sodium salt of citric acid or a hydrate thereof and a potassium salt of citric acid or a hydrate thereof;
(24) The use according to (23), in which a molar ratio between the sodium salt of citric acid and the potassium salt of citric acid is 0.85 : 1.15 to 1.15 : 0.85;
(25) The use according to any one of (1) to (24), in which the citric acid or a salt thereof is a mixture of citric acid, a sodium salt of citric acid or a hydrate thereof, and a potassium salt of citric acid or a hydrate thereof;
(26) The use according to (25), in which a molar ratio among the citric acid, the sodium salt of citric acid, and the potassium salt of citric acid is 1 : 1.7 to 2.3 : 1.7 to 2.3;
(27) The use according to (25) or (26), in which the citric acid is anhydrous citric acid, the sodium salt of citric acid is a sodium citrate dihydrate (C₆H₅Na₃O₇·2H₂O), and the potassium salt of citric acid is a potassium citrate monohydrate (C₆H₅K₃O₇·H₂O);
(28) The use according to any one of (1) to (27), in which the composition is a food;
(29) The use according to any one of (1) to (28), in which the composition is a food having a unit packaging form per meal and containing 500 mg or more of citric acid or a salt thereof;
(30) The use according to any one of (1) to (29), in which the composition is a food having a unit packaging form per meal and containing 500 mg or more and 2 g or less of citric acid or a salt thereof;
(31) The use according to any one of (1) to (30), in which the composition is a food whose packaging, container, or instruction indicates an effect of improving sleep quality, an effect of relieving stress, or an effect of reducing fatigue feeling;
(32) The use according to any one of (1) to (31), in which the composition is a food whose packaging, container, or instruction indicates an effect of increasing a ratio of time of slow wave sleep to total sleep time, decreasing the number of times of intermediate awakening, or improving good sleep feeling;
(33) The use according to any one of (1) to (32), in which the composition is administered to or ingested by a female;
(2-1) A method for improving sleep quality, including administering a composition containing an effective amount of citric acid or a salt thereof to a target in need of the improvement in sleep quality;
(2-2) The method according to (2-1), in which the improvement in sleep quality is suppression of intermediate awakening;
(2-3) The method according to (2-1) or (2-2), in which the improvement in sleep quality is suppression of early morning awakening;
(2-4) The method according to any one of (2-1) to (2-3), in which the improvement in sleep quality is improvement in good sleep feeling;
(2-5) The method according to any one of (2-1) to (2-4), in which the improvement in sleep quality is promotion of slow wave sleep;
(2-6) The method according to any one of (2-1) to (2-5), in which the improvement in sleep quality is an increase in ratio of time of non-REM sleep stage 3 to total sleep time;
(2-7) The method according to any one of (2-1) to (2-6), in which the improvement in sleep quality is promotion of slow wave sleep that decreases a ratio of time of stage 1 and increases a ratio of time of stage 3 in non-REM sleep;
(2-8) The method according to any one of (2-1) to (2-7), in which the improvement in sleep quality is improvement in sleep quality in a first sleep cycle;
(2-9) The method according to any one of (2-1) to (2-8), in which the improvement in sleep quality is an increase in a delta power value in a first sleep cycle;
(2-10) The method according to any one of (2-1) to (2-9), in which the improvement in sleep quality is promotion of formation of deep sleep in a first sleep cycle;
(2-11) The method according to any one of (2-1) to (2-10), in which the improvement in sleep quality is suppression of sleep disorder associated with frequent urination;
(2-12) The method according to any one of (2-1) to (2-11), in which the improvement in sleep quality is suppression of intermediate awakening associated with frequent urination;
(2-13) The method according to any one of (2-1) to (2-12), in which the improvement in sleep quality is improvement in refreshed feeling at the time of getting-up;
(2-14) The method according to any one of (2-1) to (2-13), in which the improvement in sleep quality is improvement in mental state;
(2-15) A method for relieving stress, including administering a composition containing an effective amount of citric acid or a salt thereof to a target in need of the relief of stress;
(2-16) A method for relieving mental stress, including administering a composition containing an effective amount of citric acid or a salt thereof to a target in need of the relief of stress;
(2-17) A method for reducing fatigue feeling, including administering a composition containing an effective amount of citric acid or a salt thereof to a target in need of reduction of fatigue feeling;
(2-18) The method according to any one of (2-1) to (2-17), in which the composition contains citric acid as an active ingredient;
(2-19) The method according to any one of (2-1) to (2-18), in which the composition contains an alkali metal salt of citric acid as an active ingredient;
(2-20) The method according to (2-19), in which the alkali metal salt of citric acid is a sodium salt of citric acid or a hydrate thereof, or a potassium salt of citric acid or a hydrate thereof;
(2-21) The method according to (2-20), in which the sodium salt of citric acid is a sodium citrate dihydrate (C₆H₅Na₃O₇·2H₂O);
(2-22) The method according to (2-20), in which the potassium salt of citric acid is a potassium citrate monohydrate (C₆H₅K₃O₇·H₂O);
(2-23) The method according to any one of (2-1) to (2-22), in which the citric acid or a salt thereof is a mixture of a sodium salt of citric acid or a hydrate thereof and a potassium salt of citric acid or a hydrate thereof;
(2-24) The method according to (2-23), in which a molar ratio between the sodium salt of citric acid and the potassium salt of citric acid is 0.85 : 1.15 to 1.15 : 0.85;
(2-25) The method according to any one of (2-1) to (2-24), in which the citric acid or a salt thereof is a mixture of citric acid, a sodium salt of citric acid or a hydrate thereof, and a potassium salt of citric acid or a hydrate thereof;
(2-26) The method according to (2-25), in which a molar ratio among the citric acid, the sodium salt of citric acid, and the potassium salt of citric acid is 1 : 1.7 to 2.3 : 1.7 to 2.3;
(2-27) The method according to (2-25) or (2-26), in which the citric acid is anhydrous citric acid, the sodium salt of citric acid is a sodium citrate dihydrate (C₆H₅Na₃O₇·2H₂O), and the potassium salt of citric acid is a potassium citrate monohydrate (C₆H₅K₃O₇·H₂O);
(2-28) The method according to any one of (2-1) to (2-27), in which the composition is a food;
(2-29) The method according to any one of (2-1) to (2-28), in which the composition is a food having a unit packaging form per meal and containing 500 mg or more of citric acid or a salt thereof;
(2-30) The method according to any one of (2-1) to (2-29), in which the composition is a food having a unit packaging form per meal and containing 500 mg or more and 2 g or less of citric acid or a salt thereof;
(2-31) The method according to any one of (2-1) to (2-30), in which the composition is a food whose packaging, container, or instruction indicates an effect of improving sleep quality, an effect of relieving stress, or an effect of reducing fatigue feeling;
(2-32) The method according to any one of (2-1) to (2-31), in which the composition is a food whose packaging, container, or instruction indicates an effect of increasing a ratio of time of slow wave sleep to total sleep time, decreasing the number of times of intermediate awakening, or improving good sleep feeling;
(2-33) The method according to any one of (2-1) to (2-32), in which the composition is administered to or ingested by a female;

Hereinafter, the present invention will be further described with reference to Examples, but the present invention is not limited thereto.

### Examples

### Example 1

25 males and females determined to be "suspected of having insomnia" with a score of 6 or more and 9 or less according to the Athens Insomnia Scale were used as targets. Each of the subjects took a placebo or a capsule containing 3 g of citrate (1392 mg of potassium citrate monohydrate, 1170 mg of sodium citrate dihydrate, and 438 mg of citric acid) every day for two weeks after breakfast and 30 minutes before going to bed. An administration rest period was set to one week. The morning after the last day of administration, survey with St. Mary's hospital sleep questionnaire (SMH) was performed on all the subjects. Among these subjects, nine subjects were put on an electroencephalograph at the time of going to bed, and their brain waves were measured until they got up. 23 subjects in SMH (average age: 38.4 years) and 8 subjects in brain wave measurement (average age: 43.5 years) were used as targets to be analyzed, and were compared with a placebo by Wilcoxon signed rank sum test. As a result, the following was confirmed.

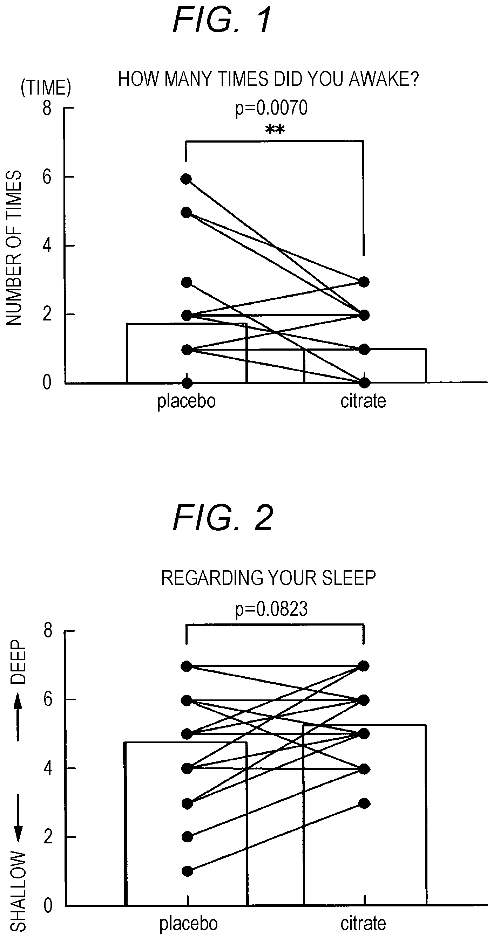

From the result of the survey with St. Mary's hospital sleep questionnaire, it was confirmed that the number of times of intermediate awakening significantly decreased in the citrate group as compared with that in the placebo group (Fig. 1).

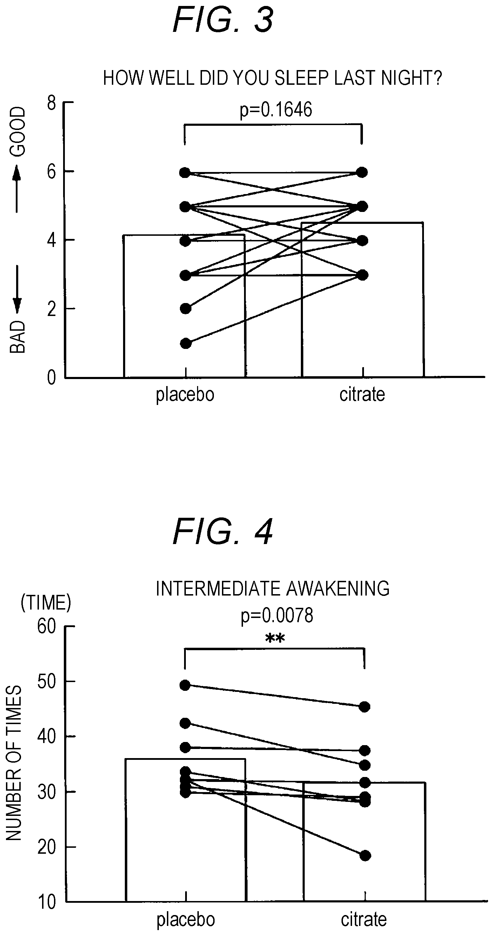

It was confirmed that deep sleep (Fig. 2) and good sleep (Fig. 3) were obtained in the citrate group as compared with sleep in the placebo group.

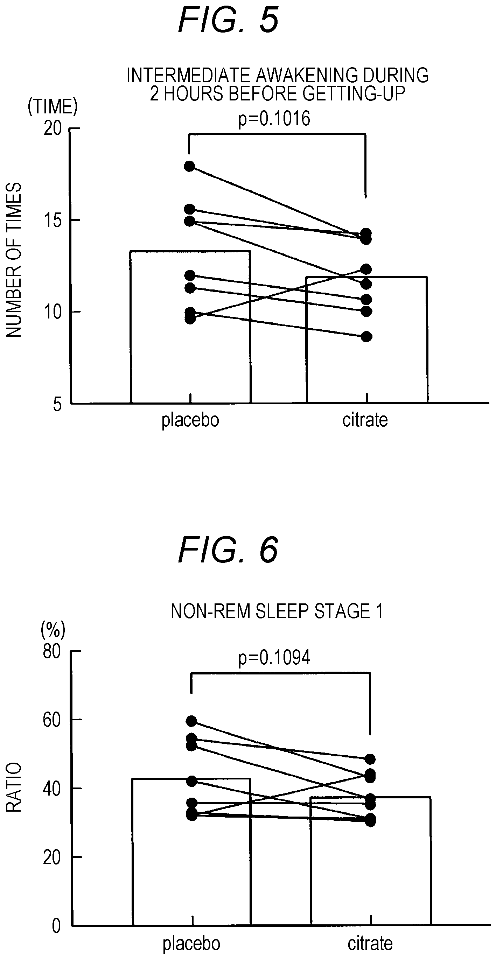

From the brain wave measurement results, it was confirmed that the number of times of intermediate awakening (Fig. 4) and the number of times of early morning awakening (Fig. 5) decreased in the citrate group as compared with those in the placebo group.

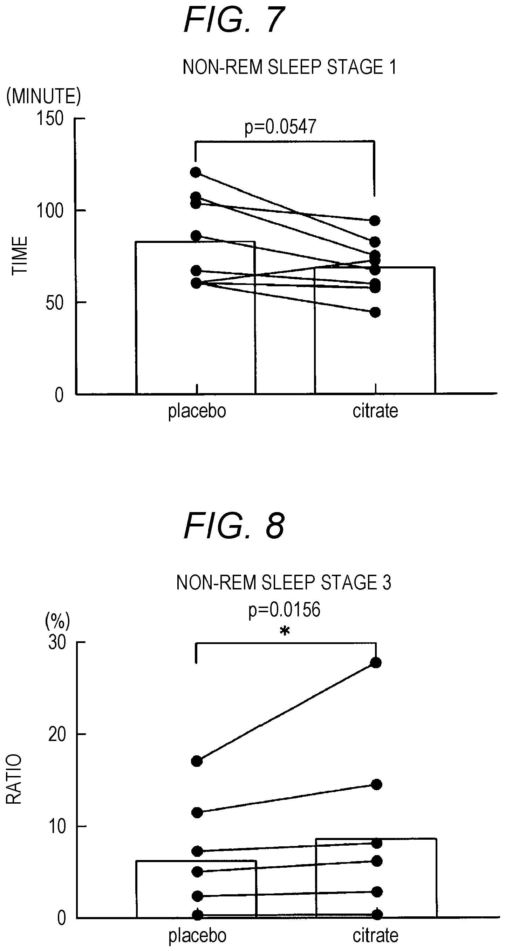

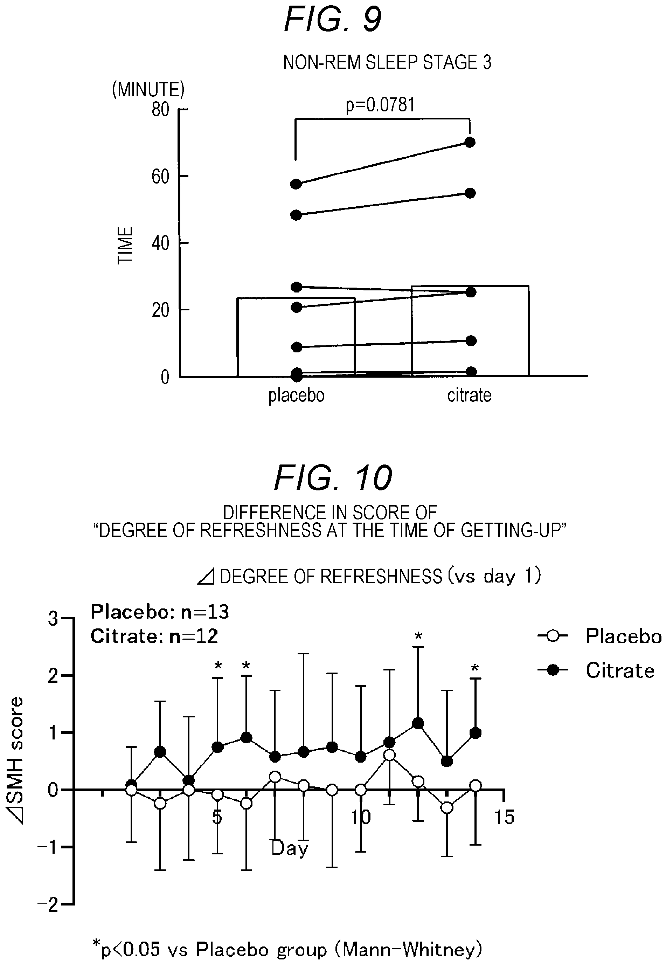

It was confirmed that a ratio (Fig. 6) and time (Fig. 7) of non-REM sleep stage 1 (shallow sleep) decreased and a ratio (Fig. 8) and time (Fig. 9) of non-REM sleep stage 3 (slow wave sleep) increased in the citrate group as compared with those in the placebo group, and a delta power value in a first sleep cycle of the citrate group increased to about 1.2 times a delta power value in a first sleep cycle of the placebo group. It has been indicated that a citrate containing hydrates of potassium citrate and sodium citrate increases the ratio and time of slow wave sleep to total sleep, decreases intermediate awakening (for example, early morning awakening), and increases sleep quality.

### Example 2

An open-label crossover test for the purpose of confirming effects of a potassium citrate/sodium citrate hydrate/citric acid blending formulation and a sodium bicarbonate formulation on a nighttime urine volume, the number of times of urination in the nighttime, and urine pH was performed on healthy males (29 to 63 years old) (the number of participating subjects: 30). The subjects received the following treatments 1) to 3) at intervals of one week or more:
1) Two tablets each containing 231.5 mg of potassium citrate (C6H5K3O7·H2O), 195.0 mg of sodium citrate hydrate (C6H5Na3O7·2H2O), and 72.5 mg of anhydrous citric acid were orally administered per day for seven days, while the daily dose was divided into three portions (morning, daytime, and evening) (group A: citric acid formulation administration group);
2) Four tablets each containing 500 mg of sodium bicarbonate were orally administered per day for seven days, while the daily dose was divided into three portions (morning, daytime, and evening) (group B: sodium bicarbonate administration group); and
3) No drug was administered for seven days (group C: control).

On the final day of drug administration in each group, urine was collected for 24 hours using a Urinemate (registered trademark) P (obtained from Sumitomo Bakelite Co., Ltd.), and time, urine volume, and urine pH were recorded for each urination. "22:00 (or later) to early morning first urine" was defined as "nighttime urine", and "second urine to 22:00" was defined as "daytime urine".

For "nighttime urine" and "daytime urine", "urine volume" and "the number of times of urination" were compared among the three groups. In addition, a relation between effects of "first urine" and "second urine" on pH and the urine volume was examined.

Note that, on the day of urine collection, the subjects' meals were managed, and influences of the meals (salinity, saccharides, protein ingestion amount, and the like) in the subjects were eliminated as much as possible.

Statistical analysis used Wilcoxon signed rank test.

Results thereof are presented in Tables 1 to 3.

**[Table 1]**

| Influence of administration of citric acid formulation or sodium bicarbonate formulation on night urine volume | | | | | | | |
|---|---|---|---|---|---|---|---|
| Group | No. | Daytime urine (mL) | Nighttime urine (mL) | 24 hour urine (mL) | Nighttime urine/24 hour urine (%) | Daytime urine-Nighttime urine (mL) | P value (daytime vs nighttime) |
| Control (C) | | 27 991 ± 417 | 554 ± 370 | 1545 ± 571 | 36 ± 15 | 438 ± 544 | 0.0004 |
| Citric acid formulation (A) | 29 | 1024 ± 345 | 421 ± 184 | 1445 ± 384 | 30 ± 11 | 603 ± 397 | < 0.0001 |
| Sodium bicarbonate formulation (B) | 26 | 1106 ± 392 | 499 ± 183 | 1605 ± 343 | 33 ± 13 | 607 ± 506 | < 0.0001 |
| P value (vs, C) | | 0.3011 (A) | 0.0999 (A) | 0.6319 (A) | 0.0126 (A) | 0.0484 (A) | |
| | | 0.0814 (B) | 0.9944 (B) | 0.0814 (B) | 0.2382 (B) | 0.1283 (B) | |
| Mean ± SD | | | | | | | |

**[Table 2]**

| Influence of administration of citric acid formulation or sodium bicarbonate formulation on number of times of urination | | | | | |
|---|---|---|---|---|---|
| Group | No. | Daytime (number of times) | Nighttime (number of times) | 24 hour urine (number of times) | P value (daytime vs nighttime) |
| Control (C) | 27 | 4.1 ± 1.2 | 2.2 ± 1.1 | 6.3 ± 1.7 | < 0.0001 |
| Citric acid formulation (A) | 29 | 4.1 ± 1.0 | 1.9 ± 0.7 | 6.0 ± 1.2 | < 0.0001 |
| Sodium bicarbonate formulation (B) | 26 | 4.2 ± 1.4 | 1.8 ± 0.6 | 6.0 ± 1.5 | < 0.0001 |
| P value (vs C) | | 0.8160 (A) | 0.1309 (A) | 0.5024 (A) | |
| | | 0.8748 (B) | 0.0898 (B) | 0.4316 (B) | |
| Mean ± SD | | | | | |

**[Table 3]**

| Influence of administration of citric acid formulation or sodium bicarbonate formulation on urine pH | | | | | | |
|---|---|---|---|---|---|---|
| Group | No. | Early morning first urine | | Early morning second urine | | P value (first urine vs second urine) |
| | | pH | ΔpH | pH | ΔpH | |
| Control (C) | 27 | 5.87 ± 0.54 | - | 6.28 ± 0.61 | - | 0.0055 |
| Citric acid formulation (A) | 29 | 6.31 ± 0.58 | 0.49 ± 0.69 | 6.91 ± 0.51 | 0.61 ± 0.64 | 0.0007 |
| Sodium bicarbonate formulation (B) | 26 | 6.79 ± 0.61 | 0.94 ± 0.67 | 7.19 ± 0.42 | 0.95 ± 0.69 | 0.0055 |
| P value | | 0.0007 (A vs C) | | < 0.0001 (A vs C) | | |
| | | < 0.0001 (B vs C) | 0.0012 | < 0.0001 (B vs C) | 0.0003 | |
| | | 0.0013 (A vs B) | | 0.0006 (A vs B) | | |
| Mean ± SD | | | | | | |

By administration of the citric acid formulation (group A), the nighttime urine volume decreased as compared with that in the control group (group C) (Table 1). In addition, by administration of the citric acid formulation (group A), the ratio of nighttime urine in the daily urine volume was reduced as compared with that in the control group (group C) (Table 1). By administration of the sodium bicarbonate formulation (group B), the nighttime urine volume decreased as compared with that in the control group (group C) (Table 1). In addition, by administration of the sodium bicarbonate formulation (group B), the ratio of nighttime urine in the daily urine volume was reduced as compared with that in the control group (group C) (Table 1).

As for the number of times of urination, by administration of the citric acid formulation (group A), the number of times of urination in the daytime was not changed, but the number of times of urination in the nighttime decreased as compared with those in the control group (group C) (Table 2). Also by administration of the sodium bicarbonate formulation (group B), the number of times of urination in the daytime was not changed, but the number of times of urination in the nighttime decreased as compared with those in the control group (group C) (Table 2) .

As for the urine pH, by administration of the citric acid formulation (group A) or the sodium bicarbonate formulation (group B), the urine pH significantly increased (alkalinized) as compared with that in the control group (group C), and the urine pH after administration of the sodium bicarbonate formulation (group B) was higher (alkalinized) than that after administration of the citric acid formulation (group A) at the tested dose (Table 3). However, the degree of the effect of alkalinizing urine by the citric acid formulation and the sodium bicarbonate formulation was not reflected in the degree of the effect of decreasing the nighttime urine volumes by these formulations. Therefore, it was suggested that the effect of decreasing the nighttime urine volume by the citric acid formulation was caused not only by alkalization of urine but also by an action of citric acid other than the alkalization of urine by the citric acid formulation (Tables 1 and 3). Similarly, the degree of the effect of alkalinizing urine by the citric acid formulation and the sodium bicarbonate formulation was not reflected in the degree of the effect of decreasing the number of times of urination in the nighttime by these formulations. Therefore, it was suggested that the effect of decreasing the number of times of urination in the nighttime by the citric acid formulation was caused not only by alkalization of urine but also by an action of citric acid other than the alkalization of urine by the citric acid formulation (Tables 2 and 3).

### Example 3

### (Testing method)

A parallel group comparative test was performed on 25 males and females (44 ± 11.4 years old) determined to be "suspected of having insomnia" with a score of 6 or more and 9 or less according to the Athens Insomnia Scale. Each of the subjects took a placebo or a capsule containing 3 g of citrate (1392 mg of potassium citrate monohydrate, 1170 mg of sodium citrate dihydrate, and 438 mg of citric acid) every day for two weeks after breakfast and 30 minutes before going to bed. The following was performed on the subjects during the test.
- St. Mary's hospital sleep questionnaire (SMH): every day from the morning after start of administration to the morning after the last day of administration
- OSA sleep questionnaire: before start of test and at end of test
- POMS2 shortened version: before start of test and at end of test
- Pulse rate: before start of test and at end of test
- Fatigue feeling VAS: during three days before start of ingestion, during three days of the first week of ingestion, and during three days of the second week of ingestion

### (Results)

From the result of the survey with St. Mary's hospital sleep questionnaire, it was confirmed that the score of "degree of refreshness at the time of getting-up" with respect to refreshness at the time of getting-up on Day 1 in the citrate group was higher (improved) than that in the placebo group on Day 5 and subsequent days (Fig. 10) .

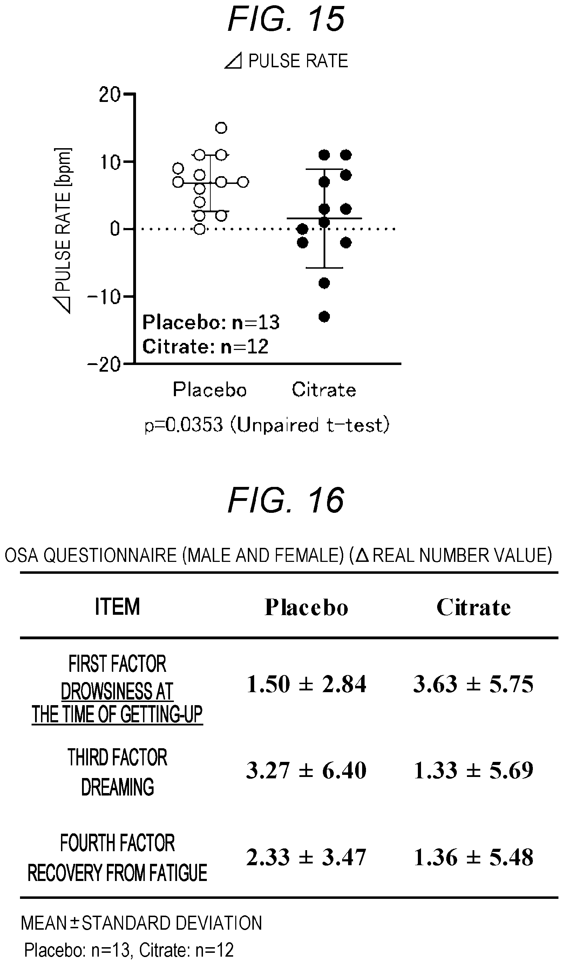

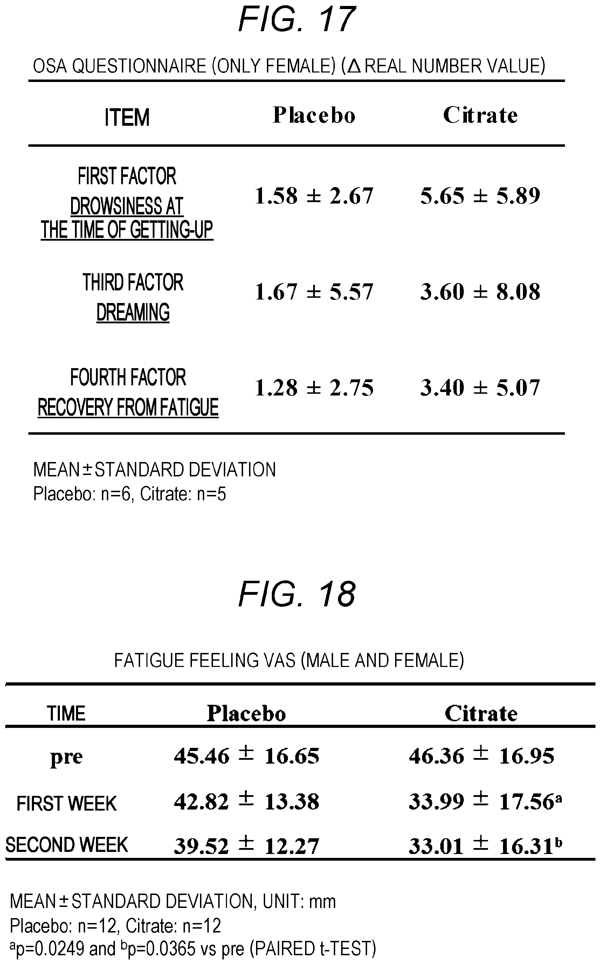

From the result of the OSA sleep questionnaire, the score of "drowsiness at the time of getting-up" in the citrate group is higher than that in the placebo group, and in females, the scores of "dreaming" and "recovery from fatigue" in the citrate group are higher than those in the placebo group in addition to "drowsiness at the time of getting-up" (Figs. 16 and 17).

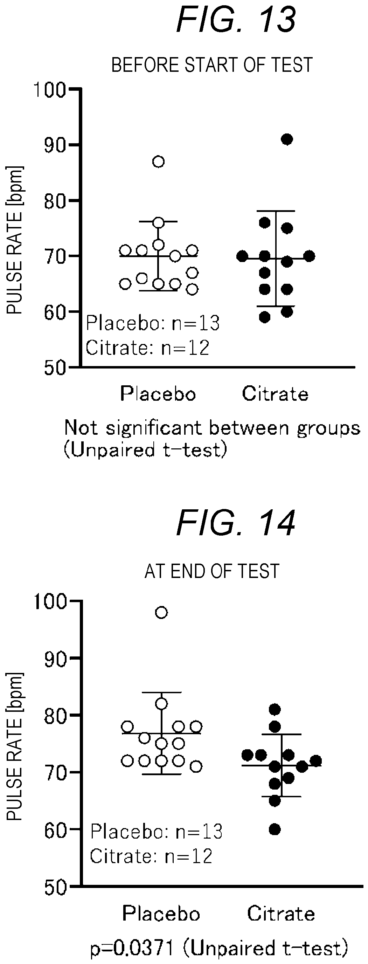

In the placebo group, the pulse rate at an end of the test was higher than that before the end of the test, but the pulse rate in the citrate group did not change (Figs. 13 to 15). It is considered that stress on participation in the test appeared as an increase in pulse rate in the placebo group, but such an increase was not exhibited in the citrate group.

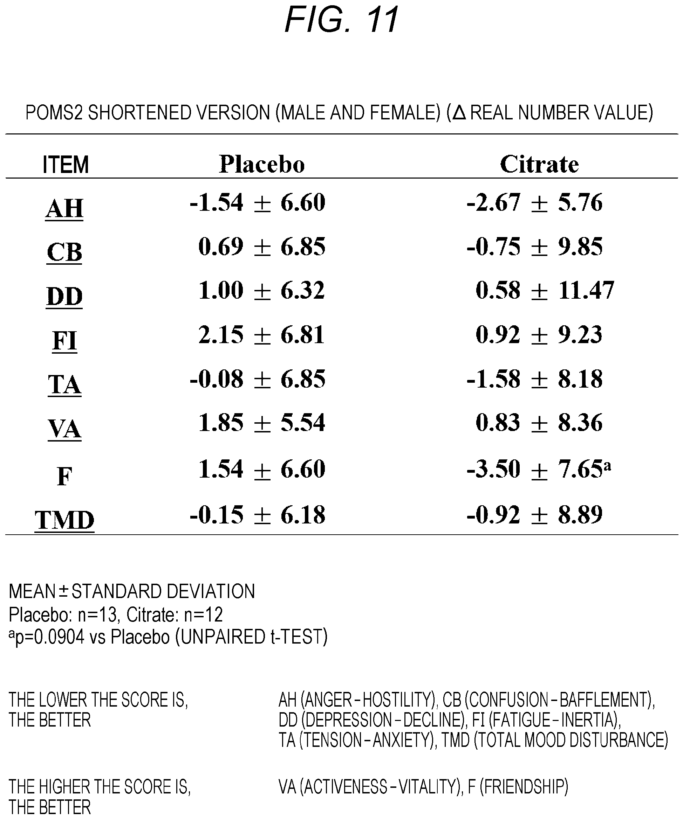

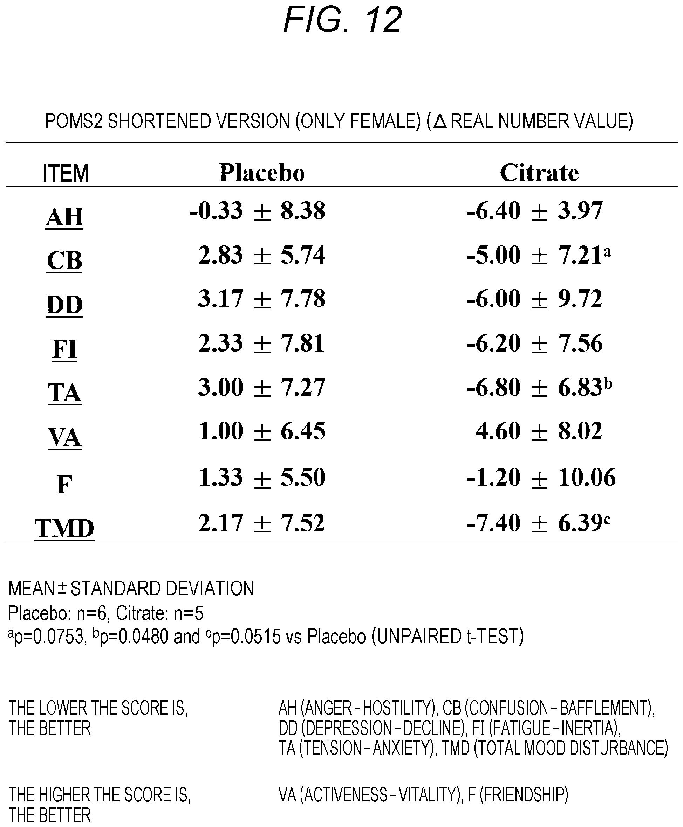

From the result of POMS2, it was confirmed that the scores regarding mood profiles such as AH (anger-hostility), CB (confusion-bafflement), DD (depression-decline), FI (fatigue-inertia), TA (tension-anxiety), VA (activeness-vitality), and TMD (total mood disturbance) were generally improved in the citrate group as compared with those in the placebo group. This effect was more apparent in females (Figs. 11 and 12). The result of POMS2 indicates that stress is relieved by citrate administration.

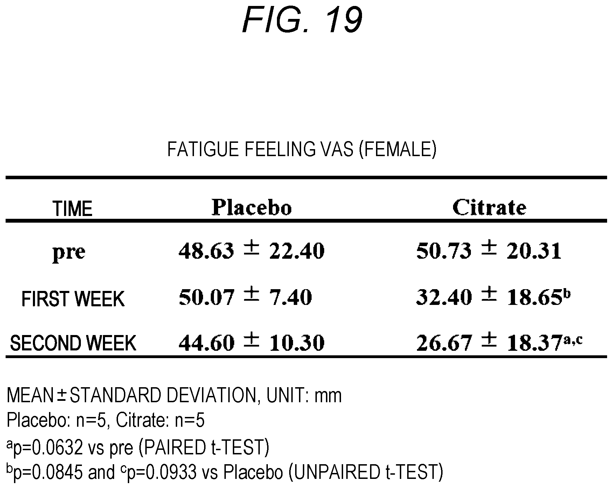

From the result of the fatigue feeling VAS, in the citrate group, fatigue feeling was significantly improved in the first week of administration, and the effect was maintained even in the second week. In addition, the effect was also larger than that of the placebo group. Furthermore, the improvement effect was more apparent in females (Figs. 18 and 19).

From the above, it has been indicated that stress is relieved, good quality sleep can be obtained, the degree of fatigue is improved, and the degree of refreshness at the time of getting-up is improved by citrate administration.

### Industrial Applicability

A food composition or a pharmaceutical composition useful for improving sleep quality, relieving stress, or reducing fatigue feeling in a mammal, particularly a human can be provided.

## Claims

1. A sleep quality improver comprising citric acid or a salt thereof as an active ingredient.

2. The sleep quality improver according to claim 1, wherein the improvement in sleep quality is suppression of intermediate awakening.

3. The sleep quality improver according to claim 1 or 2, wherein the improvement in sleep quality is suppression of early morning awakening.

4. The sleep quality improver according to any one of claims 1 to 3, wherein the improvement in sleep quality is improvement in good sleep feeling.

5. The sleep quality improver according to any one of claims 1 to 4, wherein the improvement in sleep quality is promotion of slow wave sleep.

6. The sleep quality improver according to any one of claims 1 to 5, wherein the improvement in sleep quality is an increase in ratio of time of non-REM sleep stage 3 to total sleep time.

7. The sleep quality improver according to any one of claims 1 to 6, wherein the improvement in sleep quality is promotion of slow wave sleep that decreases a ratio of time of stage 1 and increases a ratio of time of stage 3 in non-REM sleep.

8. The sleep quality improver according to any one of claims 1 to 9, wherein the improvement in sleep quality is improvement in sleep quality in a first sleep cycle.

9. The sleep quality improver according to any one of claims 1 to 8, wherein the improvement in sleep quality is an increase in a delta power value in a first sleep cycle.

10. The sleep quality improver according to any one of claims 1 to 9, wherein the improvement in sleep quality is promotion of formation of deep sleep in a first sleep cycle.

11. The sleep quality improver according to any one of claims 1 to 10, wherein the improvement in sleep quality is suppression of sleep disorder associated with frequent urination.

12. The sleep quality improver according to any one of claims 1 to 11, wherein the improvement in sleep quality is suppression of intermediate awakening associated with frequent urination.

13. The sleep quality improver according to any one of claims 1 to 12, wherein the improvement in sleep quality is improvement in refreshed feeling at the time of getting-up.

14. The sleep quality improver according to any one of claims 1 to 13, wherein the improvement in sleep quality is improvement in mental state.

15. A stress reliever comprising citric acid or a salt thereof as an active ingredient.

16. A mental stress reliever comprising citric acid or a salt thereof as an active ingredient.

17. A fatigue feeling reducer comprising citric acid or a salt thereof as an active ingredient.

18. The improver, reducer, or reliever according to any one of claims 1 to 17, comprising citric acid as an active ingredient.

19. The improver, reducer, or reliever according to any one of claims 1 to 18, comprising an alkali metal salt of citric acid as an active ingredient.

20. The improver, reducer, or reliever according to claim 19, wherein the alkali metal salt of citric acid is a sodium salt of citric acid or a hydrate thereof, or a potassium salt of citric acid or a hydrate thereof.

21. The improver, reducer, or reliever according to claim 20, wherein the sodium salt of citric acid is a sodium citrate dihydrate (C₆H₅Na₃O₇·2H₂O).

22. The improver, reducer, or reliever according to claim 20, wherein the potassium salt of citric acid is a potassium citrate monohydrate (C₆H₅K₃O₇·H₂O).

23. The improver, reducer, or reliever according to any one of claims 1 to 22, wherein the citric acid or a salt thereof is a mixture of a sodium salt of citric acid or a hydrate thereof and a potassium salt of citric acid or a hydrate thereof.

24. The improver, reducer, or reliever according to claim 23, wherein a molar ratio between the sodium salt of citric acid and the potassium salt of citric acid is 0.85 : 1.15 to 1.15 : 0.85.

25. The improver, reducer, or reliever according to any one of claims 1 to 22, wherein the citric acid or a salt thereof is a mixture of citric acid, a sodium salt of citric acid or a hydrate thereof, and a potassium salt of citric acid or a hydrate thereof.

26. The improver, reducer, or reliever according to claim 25, wherein a molar ratio among the citric acid, the sodium salt of citric acid, and the potassium salt of citric acid is 1 : 1.7 to 2.3 : 1.7 to 2.3.

27. The improver, reducer, or reliever according to claim 25 or 26, wherein the citric acid is anhydrous citric acid, the sodium salt of citric acid is a sodium citrate dihydrate (C₆H₅Na₃O₇·2H₂O), and the potassium salt of citric acid is a potassium citrate monohydrate (C₆H₅K₃O₇·H₂O).

28. The improver, reducer, or reliever according to any one of claims 1 to 27, which is a food.

29. The improver, reducer, or reliever according to any one of claims 1 to 28, which is a food having a unit packaging form per meal and containing 500 mg or more of citric acid or a salt thereof.

30. The improver, reducer, or reliever according to any one of claims 1 to 29, which is a food having a unit packaging form per meal and containing 500 mg or more and 2 g or less of citric acid or a salt thereof.

31. The improver, reducer, or reliever according to any one of claims 1 to 30, which is a food whose packaging, container, or instruction indicates an effect of improving sleep quality, an effect of relieving stress, or an effect of reducing fatigue feeling.

32. The improver, reducer, or reliever according to any one of claims 1 to 31, which is a food whose packaging, container, or instruction indicates an effect of increasing a ratio of time of slow wave sleep to total sleep time, decreasing the number of times of intermediate awakening, or improving good sleep feeling.

33. The improver, reducer, or reliever according to any one of claims 1 to 32, which is to be administered to or ingested by a female.
